# EUROPEAN PATENT SPECIFICATION

(11) **EP 3 116 483 B1**
(45) Date of publication and mention of the grant of the patent: **21.04.2021**
(21) Application number: 15761728.3
(22) Date of filing: 04.03.2015
(51) Int. Cl.: A61K 9/22, A61K 47/38, A61K 47/22

(54) **CONTROLLED RELEASE COMPOSITION AND METHOD**
ZUSAMMENSETZUNG MIT GESTEUERTER FREISETZUNG UND VERFAHREN
COMPOSITION À LIBÉRATION CONTRÔLÉE ET PROCÉDÉ

(30) Priority: 11.03.2014 US 201461951065 P; 31.10.2014 US 201462073365 P
(43) Date of publication of application: 18.01.2017
(73) Proprietor: DuPont Nutrition USA, Inc., Wilmington DE 19805 (US)
(72) Inventor: YANG, Hui S., Plainsboro, NJ 08536 (US); MA, Hua, Skillman, NJ 08558 (US); STOKES, Kevin, Hamilton Square, NJ 08690 (US)
(74) Representative: DuPont EMEA
(86) International application number: PCT/US2015/018743
(87) International publication number: WO 2015/138200

(56) References cited:
- US-A- 4 330 338
- US-A- 4 330 338
- US-A- 4 629 753
- US-A- 5 273 760
- US-A1- 2007 184 102

## Description

### FIELD OF THE INVENTION

In one aspect, the present invention is directed to a method of making a controlled release solid dosage form having an ethylcellulose coating layer, which layer comprises a coalescing agent which is an organic ester having an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters. The use of such coalescing agent permits the formation of an effective controlled release coating without the need for a further curing step after the coating process. In other aspects, this invention relates to an aqueous dispersion useful in such method; as well as to the coated dosage form produced.

### BACKGROUND OF THE INVENTION

Controlled release dosage forms are designed to provide prolonged pharmacological action after the administration of the dosage form, as compared with the administration of an immediate release dosage form. Such sustained response offers many inherent therapeutic benefits that cannot be obtained with immediate release and short acting products.

Controlled release dosage forms known in the art include coated beads, pellets or spheroids, coated capsules, coated tablets and ion exchange resins, wherein the sustained release of the active drug is realized via permeation of the active drug through a coating layer or a matrix formulation to slow down the release of the drug.

An essential characteristic of all controlled release dosage forms is the stability of the dosage forms. The stability of a pharmaceutical dosage form refers to the constancy of its physical, chemical, microbiological, therapeutic, pharmaceutical, and toxicological properties during storage in a specific container under a specific set of conditions. Stability studies are required by Good Manufacturing Practices (GMPs), the U.S.P., as well as New Drug Applications (NDAs) and Investigational New Drug Applications (INDs).

Hydrophobic polymers have been used as a film former to coat tablets, capsules, suppositories, spheroids, beads or microspheres to develop controlled release dosage forms. It is known in the prior art that these hydrophobic coatings are formulated in the form of an organic solution, pseudolatex or suspension. Since most of these polymers are insoluble in water, a polymer solution in an organic solvent is sprayed onto the individual drug forms (such as beads or tablets) and the solvent is evaporated during the coating process. However, the evaporated solvent poses environmental pollution concerns. In addition, coating formulations with organic solvents have inherent problems with regard to flammability, carcinogenicity, and safety.

For these reasons, it is desirable to use an aqueous polymer coating composition based on a latex or pseudolatex of an insoluble polymer to prepare a controlled release formulation. Among the latexes which have been successfully employed to produce desirable controlled release coatings are those based upon ethylcellulose. However, in order to increase the storage stability of such ethylcellulose coatings, additional process steps are desirably employed. Thus, for example, US Patent 7,829,148 (Li et al) discloses that the storage stability of coatings produced from aqueous dispersions of ethylcellulose or similar polymeric materials may be enhanced by a process in which the core substrate is coated under high humidity; and is then dried employing a heat treatment step. Although this process produces a desirable controlled release coating, it may require the use of specialized equipment and/or of an additional process (heating) step which needs to be monitored and increases production time. It would therefore be desirable to possess a method for producing an effective controlled release coating from aqueous dispersions of ethylcellulose which process does not require the use of such specific equipment and/or the performance of such an additional processing step.

US 4,330,338 discloses a coating composition for pharmaceutical dosage forms which comprises an aqueous dispersion of a water-insoluble polymer, e.g. ethylcellulose, an annealing agent which is a water-soluble polymer, a plasticizer, and emulsifier, e.g. sodium lauryl sulfate, and an emulsion stabilizer. The annealing agent is included to improve the properties of the resulting coating.

US 2007/0184102 discloses ethylcellulose coatings that include a permeation enhancing agent and a plasticizer to provide modulated release of a drug from film-coated dosage forms. The permeation enhancer serves to increase the release rate of the drug from the coated dosage forms. The coatings are subjected to curing after application.

US 5,273,760 discloses a method for obtaining a stable controlled release formulation by coating a solid core containing an active ingredient with an aqueous dispersion of plasticized ethylcellulose followed by curing the coated dosage form at a temperature higher than the glass transition temperature of the aqueous dispersion of ethylcellulose.

While coalescing agents are frequently employed in the production of sealants and paints, the coatings resulting from such formulations are generally designed to be impermeable. Thus, for example, US Patent 4,629,753 (Quinn) discloses compositions suitable for forming impermeable and durable coatings or films based upon aqueous polymeric dispersions (including ethylcellulose), which dispersions may optionally contain coalescing agents such as propylene glycol monostearate and the like. Somewhat similarly, US Patent 8,153,707 (Lynch et al) discloses the use of certain fatty acid esters of ethylene glycol and/or propylene glycol (including propylene glycol monolaurate) as coalescing agents in aqueous dispersions of water-insoluble polymers useful as paints, sealants, etc. Thus, it is unexpected that a controlled release coating for a dosage form could be produced from an ethylcellulose dispersion employing a coalescing agent which is an organic ester having an HLB Value of from 3 to 8.

### SUMMARY OF THE INVENTION

In one aspect, the present invention is directed to a method disclosed in claim 1, of making a controlled release solid dosage form comprising applying an aqueous suspension comprising (i) water; (ii) ethylcellulose; (iii) an ionic surfactant; and (iv) a coalescing agent which is an organic ester having an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters; to a solid core comprising an active ingredient to form a controlled release coating without the need for a further curing step after the coating process.

In another aspect, this invention is directed to a controlled release solid dosage form produced by such process.

In yet another aspect, the present invention is directed to an aqueous suspension composition as disclosed in claim 8, suitable for producing a controlled release coating without the need for a further curing step after the coating process, the composition comprising: (a) water; (b) ethylcellulose; (c) an ionic surfactant; and (d) a coalescing agent which is an organic ester having an HLB value of from 3 to 8 wherein said coalescing agent comprises a C₅-C₁₇ saturated or unsaturated aliphatic hydrocarbon moiety and is selected from the group consisting of sorbitan esters and propylene glycol monoesters.

### DETAILED DESCRIPTION OF THE INVENTION

In one aspect, the present invention is directed to a method of making a controlled release solid dosage form comprising applying an aqueous suspension comprising (i) water; (ii) ethylcellulose; (iii) an ionic surfactant; and (iv) a coalescing agent which is an organic ester having an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters; to a solid core comprising an active ingredient to form a controlled release coating without the need for a further curing step after the coating process.

Immediate release of drug is often considered to be greater than 85% of the drug released in less than 15 minutes when measured *in vitro* employing Apparatus 2 (Paddle Apparatus) in accordance with that process specified in The United States Pharmacopeial Convention ("USP"), General Chapter <711> Dissolution. Controlled release, as used herein, encompasses any release profile that is not immediate release and includes less than 85% drug released in greater than 15 minutes and 100% drug released in, for example, 2 hours, 4 hours, 6 hours or anywhere from 8 to 12 hours or longer all as measured in accordance with the USP protocol cited above. Controlled release, as used herein, means sustained release and extended release.

The solid core employed in the practice of this invention comprises an active ingredient and may be in the form of a bead, a tablet, or any other solid conventional dosage form. Among the forms which may be coated are drug-layered nonpareils, microcrystalline cellulose beads, and beads prepared by extrusion/spheronization. When the controlled release coating of the present invention is to be applied to tablets, the tablet core may comprise the active ingredient along with any pharmaceutically accepted inert pharmaceutical filler (diluent) material, including, but not limited to, sucrose, dextrose, lactose, microcrystalline cellulose, xylitol, fructose, sorbitol, mixtures thereof and the like. Also, an effective amount of any generally accepted pharmaceutical lubricant, including calcium or magnesium soaps may be added to the above-mentioned ingredients of the excipient prior to compression of the tablet core ingredients. Most preferred is magnesium stearate in an amount of about 0.5-3% by weight of the solid dosage form.

The active ingredient may comprise any pharmaceutically, therapeutically or nutraceutically active ingredient which is beneficially employed in a controlled release composition. Active ingredients which may be employed in the compositions of the present invention include both water soluble and water insoluble compounds. Illustrative, non-limiting examples of active ingredients which may be employed include antihistamines (e.g., dimenhydrinate, diphenhydramine, chlorpheniramine and dexchlorpheniramine maleate), analgesics (e.g., aspirin, codeine, morphine, dihydromorphone, oxycodone, etc.), anti-inflammatory agents (e.g., naproxyn, diclofenac, indomethacin, ibuprofen, acetaminophen, aspirin, sulindac), gastro-intestinals and anti-emetics (e.g., metoclopramide), anti-epileptics (e.g., phenytoin, meprobamate and nitrezepam), vasodilators (e.g., nifedipine, papaverine, diltiazem and nicardirine), anti-tussive, agents and expectorants (e.g., codeine phosphate), anti-asthmatics (e.g. theophylline), anti-spasmodics (e.g. atropine, scopolamine), hormones (e.g., insulin, leparin), diuretics (e.g., eltacrymic acid, bendrofluazide), anti-hypotensives (e.g., propranolol, clonidine), bronchodilators (e.g., albuterol), anti-inflammatory steroids (e.g., hydrocortisone, triamcinolone, prednisone), antibiotics (e.g., tetracycline), antihemorrhoidals, hypnotics, psychotropics, antidiarrheals, mucolytics, sedatives, decongestants, laxatives, antacids, vitamins, stimulants (including appetite suppressants such as phenylpropanolamine). The above list is not intended to be exclusive.

The aqueous suspensions of this invention, which are suitable for producing a controlled release coating without the need for a further curing step after the coating process and which are employed in the process of this invention, comprise ethylcellulose; an ionic surfactant; and a coalescing agent which is an organic ester an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters.

Typical aqueous dispersions can contain 20-40 weight percent ethylcellulose. Commercially available ethylcellulose aqueous dispersions are, for example, available from FMC Corporation and sold under the name Aquacoat® ECD and from Colorcon sold under the name Surelease®. Aquacoat® ECD is an aqueous dispersion containing 24.5%-29.5% by weight ethylcellulose, 0.9-1.7% by weight sodium lauryl sulfate and 1.7-3.3% by weight of cetyl alcohol.

The coalescing agent employed in the present invention possesses an HLB Value of from 3 to 8. Preferably, the coalescent agent possesses an HLB Value of from 3.5 to 7, most preferably the coalescent agent possesses an HLB Value of from 4 to 5. The term "HLB Value" refers to the hydrophilic lipophilic balance of ampiphilic molecules that contain both such groups. The hydrophilic lipophilic balance (HLB) value is used as a measure of the ratio of these groups. HLB values are calculated for nonionic molecules, which values range from 0-20. Compounds with an HLB Value above 10 have an affinity for water (hydrophilic); and those with an HLB value below 10 have an affinity of oil (lipophilic). Ionic surfactants have recently been assigned relative HLB values, allowing the range of numbers to extend to 60.

The coalescing agent employed in the practice of this invention is an organic ester having the formula RCOOR¹ wherein R is a C₅-C₁₇ hydrocarbon moiety, and R¹ is an organic moiety having sufficient hydrophilicity such that said ester possesses an HLB value of from 3 to 8. R may be aliphatic or comprise an aromatic group, and may be saturated or unsaturated. Generally, R is a C₅-C₁₇ saturated or unsaturated aliphatic hydrocarbon moiety. As is employed herein, the term "organic moiety" refers to a carbon-based moiety which may be substituted with one or more hydrogen, oxygen, sulfur, or nitrogen atoms.

One class of preferred coalescing agents are propylene glycol monoesters of the formula RCOOCH₂CH(CH₃)OH wherein R is a C₅-C₁₇ saturated or unsaturated aliphatic hydrocarbon moiety. Preferred coalescing agents of this type include propylene glycol monocaprylate and propylene glycol monolaurate, with propylene glycol monolaurate being particularly preferred. As a practical matter, when a propylene glycol monoester is employed, such compound is typically in a mixture with its corresponding diester. Preferably, when the coalescing agent comprises propylene glycol monolaurate such mixture contains at least 40% by weight of the monoester; more preferably such mixture contains at least 90% by weight of the monoester.

A second class of preferred coalescing agents are sorbitan esters wherein R is a C₅-C₁₇ saturated or unsaturated aliphatic hydrocarbon, particularly sorbitan monooleate.

The coalescing agent is typically present in an amount between 4% and 30%, preferably between 9% and 15%, by weight, based upon the total weight of ethylcellulose polymer.

The surfactant employed in such aqueous suspensions may be any ionic surfactant which is pharmaceutically acceptable and which effectively maintains the stability of the dispersion during storage. Although such surfactant may be a cationic or an anionic surfactant, typically an anionic surfactant is employed. Illustrative of the anionic surfactants that may be employed are alkali metal and ammonium soaps (e.g., sodium, potassium or ammonium salts of long chain fatty acids such as oleic, stearic and ricinoleic acid); divalent and trivalent metal soaps; amine soaps; alkyl sulfates; and alkyl phosphates. See P. Muthuprasana et al; Basic and Potential Applications of Surfactants -A Review, Int. J. PharmaTech. Res. 2009, 1(4). Preferred surfactants include alkyl sulfates, with sodium lauryl sulfate being particularly preferred.

The surfactant is employed in amounts sufficient to maintain the stability of the suspension, and typically comprises between 0.2% and 2%, preferably between 0.9% and 1.7%, by weight based upon the total weight of the suspension.

It is preferred that the aqueous polymer coating composition used in the present invention include an effective amount of a suitable plasticizer, as it has been found that the use of a plasticizer with the aqueous dispersion will further improve the physical properties of the film. Examples of suitable plasticizers include water insoluble plasticizers such as dibutyl sebacate, diethyl phthalate, triethyl citrate, tributyl citrate, propylene glycol, polyethylene glycol and triacetin, although it is possible that other water-insoluble plasticizers (such as acetylated monoglycerides, phthalate esters, castor oil, etc.) may be used. Triethyl citrate is an especially preferred plasticizer. The plasticizer is typically present in an amount between 15% and 40%, preferably between 20% and 30%, by weight, based upon the total weight of ethylcellulose polymer.

The aqueous suspensions employed in the practice of the present invention preferably contain, in addition to the film-former, plasticizer, and solvent system (i.e., water), a colorant to provide elegance and product distinction. Color may be added to the solution of the therapeutically active agent instead of, or in addition to, the aqueous polymer coating composition. For example, color may be added to AQUACOAT® ECD ethylcellulose dispersion via the use of alcohol or propylene glycol based color dispersions, milled aluminum lakes and opacifiers such as titanium dioxide, by adding color with shear to a water soluble polymer solution and then using low shear when adding to the plasticized AQUACOAT® ECD ethylcellulose dispersion. Alternatively, any suitable method of providing color to the formulations of the present invention may be used.

The aqueous suspensions may be applied to the solid core employing equipment well known one of skill in the art. Thus, the aqueous suspension may be coated onto beads employing a bottom spray fluid bed coater with a Wurster insert. Top spray and tangential spray fluid beds and modified coating pans can also be used for bead coating. Side-vented and conventional coating pans can be used for tablet coating.

It is essential that the latex or pseudolatex particles do not aggregate, flocculate or coagulate in the dispersion prior to or during application, e.g., by spraying or fluidization onto the substrate, as loosely-packed or adherent particles resistant to capillary forces disrupt the mechanism of film formation which relies upon close packing of the particles on the substrate surface followed by sintering or coalescing into a coherent film.

The solid dosage forms of this invention are formed by coating the solid core with the aqueous dispersion as is described above. The aqueous suspension is applied in an amount such that a coating having the desired release rate is obtained. This coating thickness is selected based upon a number of factors including the particular active ingredient involved, the desired release rate, the particle size and shape of the uncoated substrate, the smoothness of its surface and the like. In general, the thickness of the formed film should be 5 microns or greater. Films less than 5 microns thick may have insufficient film strength and integrity and this may cause the film properties to change over time. Although there is no specific upper limit, when the film is too thick the release rate may be excessively slowed. Typically, the coating will be between 50 and 250 µm thick.

The aqueous suspension employed in the practice of the present invention forms an effective controlled release dosage form without any specific humidity requirement or additional heating step.

### EXAMPLES

The following examples are provided to illustrate the invention in accordance with the principles of this invention.

### Example 1

### Effect of Propylene Glycol Monoester on Minimum Film Formation Temperature ("MFFT")

Several aqueous suspensions based upon AquaCoat ECD-30 were prepared by adding the coalescing agent indicated in the weights indicated in Table 1 below (based on weight of ethylcellulose solids) along with 24% by weight of triethyl citrate ("TEC") (based on weight of the ethylcellulose polymer). Deionized water was added to dilute the suspension to 15% solids.

The MFFT of such suspensions was tested with MFFT-bar model 90. The MFFT bar was preset and the desired temperature range was selected depending on predicted MFFT value. Ranges 3 (5 °C to 18 °C), 4 (15°C-33°C) or range 5 (23°C to 50°C) were used in this study. The formulation was applied using a 400 micron cube applicator from warm side to cold side, and the MFFT value was read after the film was formed.

The following coalescing agents were evaluated:
PGML - propylene glycol monolaurate; Lauroglycol™ 90 (Gattefosse) (HLB 4.3)
PGMC - propylene glycol monocaprylate; Capryol™ 90 (Gattefosse) (HLB 6)
PGML/PGDL - a mixture containing 51.5% propylene glycol monolaurate and 48.5% propylene glycol dilaurate.

The results of such testing are summarized in Table 1 below.

**Table 1**

| | MFFT (°C) | | |
|---|---|---|---|
| %Aid | PGML | PGMC | PGML/PGDL (51.5% PGML) |
| 0 | >23 | >23 | |
| 4.1 | 9.3/9.70 | 11.1/10.8 | |
| 8.5 | 10.8 | 16.7 | |
| 18.5 | 9 | 16.7 | 8.9 |

The above data indicates that all three suspensions which comprised a coalescing agent comprising a propylene glycol monoester exhibited a reduced MFFT. The formulations were homogeneous and produced a smooth film when applied at temperature higher than the MFFT.

### Example 2

### Effect of Other Similar Chemicals on MFFT

Employing the process of Example 1, several suspensions were prepared employing similar compounds as coalescing agents. Such compounds were added at a rate of 9% by weight (based on weight of ethylcellulose solids). The compounds evaluated were PGDL (propylene glycol dilaurate; HLB 2)), GMC (glyceryl monocaprylate, HLB 8.3), GML (glyceryl monooleate, HLB 2.8) and TGDS (triglycerol diisosterarate HLB 10-13). While PGDL didn't effectively decrease the MFFT of the formulation, GMC, GML and TGDS didn't have good compatibility with the formulation. Sticky residue was found in the formulation even after mixing for extended time. The film on the MFFT bar was not smooth or transparent.

### Example 3

### Effect of PGML on Theophylline Release Rate

Theophylline pellets (70% theophylline; Spansules PharmaTech) were coated with 24% TEC plasticized Aquacoat® ECD coating formulations with or without coalescing agents. The TEC use level was based on the weight of ethylcellulose polymer. KolliCoat® IR was used as pore former. The KolliCoat® IR : ethylcellulose weight ratio was kept at 15/85. The PGML was added into the AquaCoat ECD-30 when the coating formulations were made.

The coating process conditions were inlet temperature 65°C, spray rate 10.0 g/min, dew point 10°C, air flow 65 m³/h, atomization pressure 2.0 bar. The coating conditions were the same with or without coalescing agent. While the 8.9% PGML formulation was coated onto Theophylline pellets without any abnormal observations, 27% PGML formulation had some stickiness during coating process.

The dissolution profile of the pellets was measured with USP apparatus 1 with 100 rpm agitation speed and 900 mL volume in 0.05 M phosphate buffer at pH 7.5 with UV absorbance at 271 nm. Table 2 shows the Theophylline dissolution profile of the uncured pellets (without post cure) and cured pellets (post cure at 60 °C for 2 hours under 75% relative humidity). Specifically, the percentage of drug released from the pellets is listed.

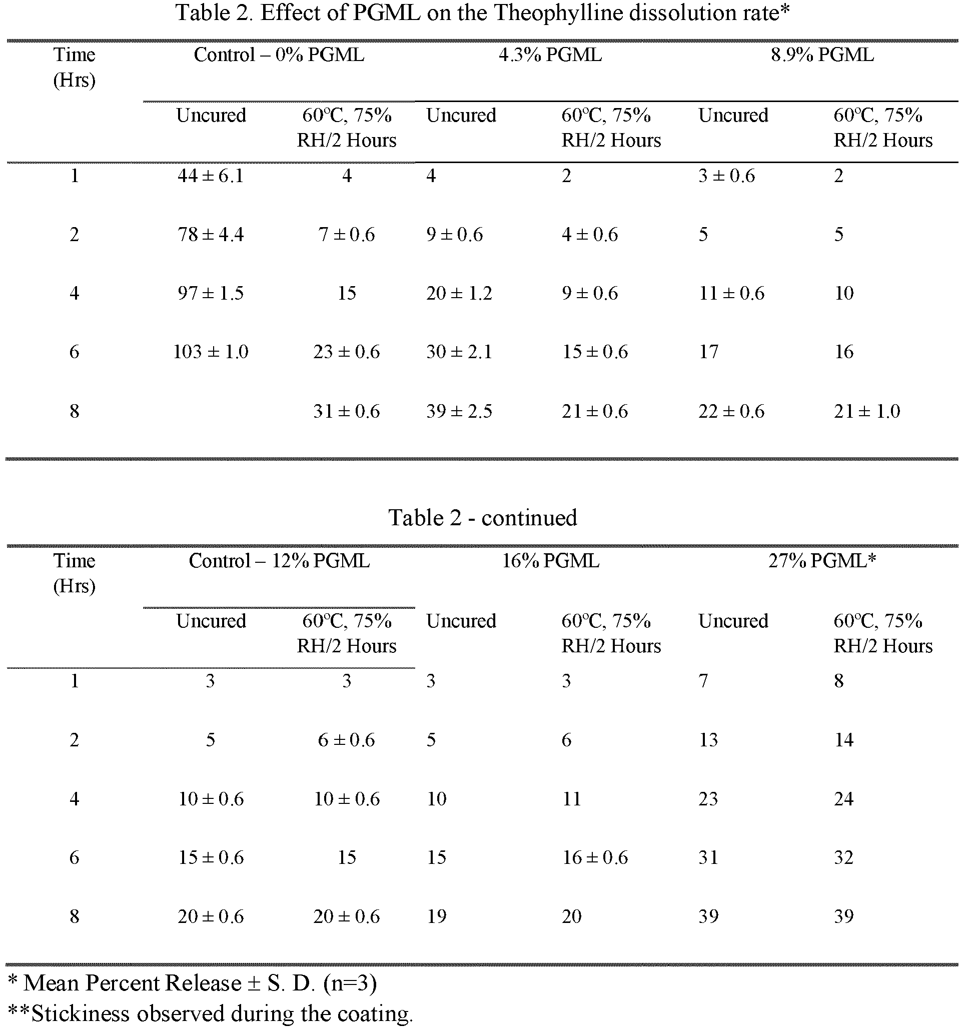

The above results indicate that the compositions of the present invention exhibit desirable controlled release properties without the need for high humidity and/or high temperature curing.

### Example 4

### Effect of Sorbitan Monooleate on Theophylline release rate

Employing the process and materials of Example 3, tablets were produced comprising 12% sorbitan monooleate as the coalescing agent (rather than PGML). The percentage of drug released for both uncured and cured (60°C for 2 hours) was measured as described in Example 3. The results of such testing are summarized in Table 3.

**Table 3**

| Mean Percent Release ± S.D. (n=3) | | |
|---|---|---|
| Time (Hours) | Uncured | Cured |
| 1 | 6 | 4 ± 0.6 |
| 2 | 12 ± 0.6 | 9 ± 0.6 |
| 4 | 21 ± 0.6 | 17 |
| 6 | 29 ± 1.0 | 24 ± 0.6 |
| 8 | 37 ± 1.0 | 30 ± 1.0 |

The above results indicate that the compositions of the present invention exhibit desirable controlled release properties without the need for high humidity and/or high temperature curing.

### Example 5

### Effect of PGML on Diltiazem release rate

Diltiazem HCl pellets (60% diltiazem HCL; Ria International) were coated with Aquacoat® ECD with or without coalescing agents and plasticized with 24% TEC based on ethylcellulose solids. The coating process conditions were inlet temperature 65°C, spray rate 10.0 g/min, dew point 10°C, air flow 65 m³/h, atomization pressure 2.0 bar. Coated pellets were cured at 60°C for 2 hours without humidity control. The inlet temperature was reduced to 50° C- 55 C° to avoid stickiness when high amount of coalescing agent was used. The dissolution profile of Diltiazem HCl pellets was measured with USP apparatus 1 with 100 rpm agitation speed and 900 mL volume in deionized water with UV absorbance at 237 nm. The results of such testing are summarized in Table 4 below:

**Table 4. Effect of PGML on Diltiazem HCl release rate***

| Time (Hrs) | Control - 0% PGML | | 8.9% PGML | |
|---|---|---|---|---|
| | (Uncured) | (60°C/2 hours) | (Uncured) | (60°C/2 hours) |
| 1 | 76 ± 1.5 | 2 ± 0.6 | 7 ± 0.7 | 4 ± 0.6 |
| 2 | 87 ± 1.2 | 13 ± 1.2 | 17 | 12 ± 1.7 |
| 4 | 93 | 36 ± 1.7 | 40 ± 0.7 | 31 ± 2.1 |
| 6 | 96 | 53 ± 1.0 | 55 ± 0.7 | 47 ± 2.1 |
| 8 | 99 | 65 ± 1.0 | 66 | 59 ± 2.0 |

| | | | | |
|---|---|---|---|---|
| * Mean Percent Release ± S. D. (n=3) | | | | |

The above results indicate that the compositions of the present invention exhibit desirable controlled release properties without the need for high humidity and/or high temperature curing.

### Example 6

### Effect of Sorbitan Monooleate on Diltiazem release rate

Pellets were produced employing the process and materials described in Example 5, except that 12% sorbitan monooleate was employed as the coalescing agent (rather than PGML). The percentage of drug released for both uncured and cured (60°C for 2 hours; and at 60°C for 2 hours at 75% relative humidity) was measured as described in Example 5. The results of such testing are summarized in Table 5 below.

**Table 5**

| Mean Percent Release ± S.D. (n=3) | | | |
|---|---|---|---|
| Time (Hours) | Uncured | Cured (2 hours at 60°C) | Cured (2 hrs. 60°C at 75% RH) |
| 1 | 1 ± 0.6 | 1 | 27 ± 3.1 |
| 2 | 6 ± 0.6 | 4 ± 1.2 | 36 ± 4.2 |
| 4 | 24 ± 1.5 | 15 ± 1.5 | 47 ± 3.6 |
| 6 | 40 ± 3.1 | 30 ± 1.7 | 55 ± 2.1 |
| 8 | 53 ± 3.2 | 44 ± 2.1 | 60 ± 1.5 |

### Example 7

### Effect of propylene glycol monolaurate and dilaurate mixture on Theophylline release rate

The process of Example 3 was repeated except a mixture of PGML and PGDL (propylene glycol dilaurate) comprising 51.5 weight percent PGML and 48.5 weight percent PGDL was used instead of PGML which contains about 94 weight percent PGML. The total weight of PGML and PGDL employed was 8.9% based on the ethylcellulose content The results of such evaluation are summarized in Table 6 below.

**Table 6. Effect of PGML and PGDL on Theophylline dissolution profile***

| Time (hrs) | 94% PGML /6% | PGDL mixture | 51.5% PGML /48.5% | PGDL mixture |
|---|---|---|---|---|
| | uncured | cured | uncured | cured |
| 1 | 3 ± 0.6 | 2 | 2 ± 0.6 | 2 |
| 2 | 5 | 5 | 4 | 4 |
| 4 | 11 ± 0.6 | 10 | 9 ± 0.6 | 7 ± 0.6 |
| 6 | 17 | 16 | 13 | 11 ± 0.6 |
| 8 | 22 ± 0.6 | 21 ± 1.0 | 18 ± 0.6 | 15 |

| | | | | |
|---|---|---|---|---|
| * Mean Percent Release ± S. D. (n=3) | | | | |

The results above demonstrate that the 51.5%/48.5% PGML/PGDL mixture worked well.

### Example 8

### Effect of PGMC on Theophylline release rate

The process of Example 3 was conducted with propylene glycol monocaprylate (PGMC) instead of PGML. The results of such evaluation are summarized in Table 7.

**Table 7. Effect of PGMC on Theophylline release rate***

| Time (Hrs) | Control - 0% PGMC | | 8.9% PGMC | | 12% PGMC | |
|---|---|---|---|---|---|---|
| | Uncured | 60°C, 75% RH/2 Hours | Uncured | 60°C, 75% RH/2 Hours | Uncured | 60°C, 75% RH/2 Hours |
| 1 | 44 ± 6.1 | 4 | 3 | 2 ± 0.6 | 2 ± 0.6 | 2 |
| 2 | 78 ± 4.4 | 7 ± 0.6 | 6 | 4 ± 0.6 | 5 ± 0.6 | 4 |
| 4 | 97 ± 1.5 | 15 | 13 | 8 ± 0.6 | 10 ± 1.2 | 8 |
| 6 | 103 ± 1.0 | 23 ± 0.6 | 20 | 13 ± 0.6 | 16 ± 1.2 | 12 |
| 8 | | 31 ± 0.6 | 27 ± 0.6 | 18 ± 0.6 | 20 ± 1.5 | 16 ± 0.6 |

| | | | | | | |
|---|---|---|---|---|---|---|
| * Mean Percent Release ± S. D. (n=3) | | | | | | |

The above results demonstrate that PGMC was able to improve the coalescing of the ECD formulations and resulted acceptable results as controlled release coatings without further curing process.

### Example 9

### Stability of Mixture of ECD-30 with PGML

AquaCoat ECD-30 was blended with 8.9% PGML (based upon the weight of ethylcellulose solids in the dispersion) and aged for 3 months at room conditions (20-25° C) and elevated conditions (40° C; 75% RH). After such storage, TEC and other ingredients were added and the mixture diluted to a 15% solid concentration. Theophylline pellets were coated and resultant solid dosage forms evaluated following the procedures set forth in Example 3. The results of such evaluation are summarized in Table 8.

**Table 8**

| Time (Hrs) | 8.9%PGML Uncured | | | 8.9% PGML Cured 60°C, 75% RH/2 Hours | | |
|---|---|---|---|---|---|---|
| | Initial | RT /3 months | 40°C 75%RH /3 months | Initial | RT /3 months | 40 °C 75%RH /3 months |
| 1 | 3 ± 0.6 | 2 | 3 | 2 | 2 | 3 ± 0.6 |
| 2 | 5 | 4 ± 0.6 | 7 | 5 | 4 | 5 |
| 4 | 11 ± 0.6 | 9 | 14 ± 1.0 | 10 | 8 | 10 ± 0.6 |
| 6 | 17 | 14 | 21 ± 1.0 | 16 | 13 | 14 ± 1.2 |
| 8 | 22 ± 0.6 | 19 | 28 ± 1.2 | 21 ± 1.0 | 16 ± 0.6 | 19 ± 1.2 |

| | | | | | | |
|---|---|---|---|---|---|---|
| * Mean Percent Release ± S. D. (n=3) | | | | | | |

The above results show that aqueous suspensions of this invention are storage stable for extended periods of time, even under high humidity and elevated temperature conditions.

## Claims

1. A method of making a controlled release solid dosage form comprising applying an aqueous suspension comprising (i) water; (ii) ethylcellulose; (iii) an ionic surfactant; and (iv) a coalescing agent which is an organic ester having an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters; to a solid core comprising an active ingredient to form a controlled release coating without the need for a further curing step after the coating process.

2. The method of claim 1 wherein the coalescing agent has an HLB Value of from 4 to 5.

3. The method of claim 1 or 2 wherein the coalescing agent is selected from the group consisting of propylene glycol monolaurate, sorbitan monooleate and propylene glycol monocaprylate.

4. The method of any of the preceding claims wherein the coalescing agent is present in an amount between 4% and 30% by weight, based upon the total weight of the ethylcellulose.

5. The method of any of the preceding claims wherein the surfactant is an anionic surfactant.

6. The method of any of the preceding claims wherein the aqueous suspension further comprises a plasticizer.

7. A solid dosage form produced by the method of any of the preceding claims.

8. An aqueous suspension composition suitable for producing a controlled release coating without the need for a further curing step after the coating process, the composition comprising:
a) water;
b) ethylcellulose;
c) an ionic surfactant; and
d) a coalescing agent which is an organic ester an HLB value of from 3 to 8, wherein said coalescing agent comprises a C₅-C₁₇ saturated or unsaturated aliphatic hydrocarbon moiety and is selected from the group consisting of sorbitan esters and propylene glycol monoesters.

9. The composition of claim 8 wherein the coalescing agent has an HLB Value of from 4 to 5.

10. The composition of claim 9 wherein the coalescing agent is selected from the group consisting of propylene glycol monolaurate, sorbitan monooleate and propylene glycol monocaprylate.

11. The composition of any of the preceding composition claims wherein the coalescing agent is present in an amount between 4% and 30% by weight, based upon the total weight of the ethylcellulose.

12. The composition of any of the preceding composition claims wherein the surfactant is an anionic surfactant.

13. The composition of any of the preceding composition claims, wherein the aqueous suspension further comprises a plasticizer.

## Patentansprüche

1. Verfahren zum Herstellen einer festen Darreichungsform geregelter Freisetzung, umfassend Auftragen einer wässrigen Suspension umfassend (i) Wasser, (ii) Ethylcellulose, (iii) ein ionisches Tensid und (iv) ein Koalesziermittel, das ein organischer Ester ist, der einen HLB-Wert von 3 bis 8 aufweist, wobei das Koalesziermittel einen gesättigten oder ungesättigten aliphatischen C₅-C₁₇-Kohlenwasserstoffanteil umfasst und aus der Gruppe ausgewählt ist bestehend aus Sorbitanestern und Propylenglycolmonoestern, auf einen festen Kern umfassend einen Wirkstoff, um eine Beschichtung geregelter Freisetzung ohne die Notwendigkeit eines weiteren Aushärtungsschritts nach dem Beschichtungsvorgang zu bilden.

2. Verfahren nach Anspruch 1, wobei das Koalesziermittel einen HLB-Wert von 4 bis 5 aufweist.

3. Verfahren nach Anspruch 1 oder 2, wobei das Koalesziermittel aus der Gruppe ausgewählt ist bestehend aus Propylenglycolmonolaurat, Sorbitanmonooleat und Propylenglycolmonocaprylat.

4. Verfahren nach einem der vorhergehenden Ansprüche, wobei das Koalesziermittel in einer Menge zwischen 4 und 30 Gew.-%, auf das Gesamtgewicht der Ethylcellulose bezogen, vorliegt.

5. Verfahren nach einem der vorhergehenden Ansprüche, wobei das Tensid ein anionisches Tensid ist.

6. Verfahren nach einem der vorhergehenden Ansprüche, wobei die wässrige Suspension ferner einen Weichmacher umfasst.

7. Feste Darreichungsform, die durch das Verfahren nach einem der vorhergehenden Ansprüche hergestellt wird.

8. Wässrige Suspensionszusammensetzung, die zum Herstellen einer Beschichtung regulierter Freisetzung ohne die Notwendigkeit eines weiteren Aushärtungsschritts nach dem Beschichtungsvorgang geeignet ist, wobei die Zusammensetzung Folgendes umfasst:
a) Wasser;
b) Ethylcellulose;
c) ein ionisches Tensid; und
d) ein Koalesziermittel, das ein organischer Ester ist, der einen HLB-Wert von 3 bis 8 aufweist, wobei das Koalesziermittel einen gesättigten oder ungesättigten aliphatischen C₅-C₁₇-Kohlenwasserstoffanteil umfasst und aus der Gruppe ausgewählt ist bestehend aus Sorbitanestern und Propylenglycolmonoestern.

9. Zusammensetzung nach Anspruch 8, wobei das Koalesziermittel einen HLB-Wert von 4 bis 5 aufweist.

10. Zusammensetzung nach Anspruch 9, wobei das Koalesziermittel aus der Gruppe ausgewählt ist bestehend aus Propylenglycolmonolaurat, Sorbitanmonooleat und Propylenglycolmonocaprylat.

11. Zusammensetzung nach einem der vorhergehenden Zusammensetzungsansprüche, wobei das Koalesziermittel in einer Menge zwischen 4 und 30 Gew.-%, auf das Gesamtgewicht der Ethylcellulose bezogen, vorliegt.

12. Zusammensetzung nach einem der vorhergehenden Zusammensetzungsansprüche, wobei das Tensid ein anionisches Tensid ist.

13. Zusammensetzung nach einem der vorhergehenden Zusammensetzungsansprüche, wobei die wässrige Suspension ferner einen Weichmacher umfasst.

## Revendications

1. Procédé pour la fabrication d'une forme galénique solide à libération contrôlée comprenant l'application d'une suspension aqueuse comprenant (i) de l'eau; (ii) de l'éthylcellulose; (iii) un tensioactif ionique; et (iv) un agent coalescent qui est un ester organique ayant une valeur HLB de 3 à 8, dans lequel ledit agent coalescent comprend une fraction hydrocarbure C₅-C₁₇ aliphatique saturée ou insaturée et est choisi dans le groupe constitué d'esters de sorbitan et de monoesters de propylène glycol; sur un cœur solide comprenant un ingrédient actif pour former un enrobage à libération contrôlée sans le besoin d'une étape de cuisson supplémentaire après le procédé d'enrobage.

2. Procédé selon la revendication 1, dans lequel l'agent coalescent a une valeur HLB de 4 à 5.

3. Procédé selon la revendication 1 ou 2, dans lequel l'agent coalescent est choisi dans le groupe constitué de monolaurate de propylène glycol, de monooléate de sorbitan et de monocaprylate de propylène glycol.

4. Procédé selon l'une quelconque des revendications précédentes, dans lequel l'agent coalescent est présent en une quantité entre 4% et 30% en poids, sur la base du poids total de l'éthylcellulose.

5. Procédé selon l'une quelconque des revendications précédentes, dans lequel le tensioactif est un tensioactif anionique.

6. Procédé selon l'une quelconque des revendications précédentes, dans lequel la suspension aqueuse comprend en outre un plastifiant.

7. Forme galénique solide produite par le procédé selon l'une quelconque des revendications précédentes.

8. Composition de suspension aqueuse appropriée pour la production d'un enrobage à libération contrôlée sans le besoin d'une étape de cuisson supplémentaire après le procédé d'enrobage, la composition comprenant:
a) de l'eau;
b) de l'éthylcellulose;
c) un tensioactif ionique; et
d) un agent coalescent qui est un ester organique ayant une valeur HLB de 3 à 8, dans laquelle ledit agent coalescent comprend une fraction hydrocarbure C₅-C₁₇ aliphatique saturée ou insaturée et est choisi dans le groupe constitué d'esters de sorbitan et de monoesters de propylène glycol.

9. Composition selon la revendication 8, dans laquelle l'agent coalescent a une valeur HLB de 4 à 5.

10. Composition selon la revendication 9, dans laquelle l'agent coalescent est choisi dans le groupe constitué de monolaurate de propylène glycol, de monooléate de sorbitan et de monocaprylate de propylène glycol.

11. Composition selon l'une quelconque des revendications de composition précédentes, dans laquelle l'agent coalescent est présent dans une quantité entre 4% et 30% en poids, sur la base du poids total de l'éthylcellulose.

12. Composition selon l'une quelconque des revendications de composition précédentes, dans laquelle le tensioactif est un tensioactif anionique.

13. Composition selon l'une quelconque des revendications de composition précédentes, dans laquelle la suspension aqueuse comprend en outre un plastifiant.
